# EUROPÄISCHE PATENTSCHRIFT

(11) **EP 1 505 917 B1**
(45) Veröffentlichungstag und Bekanntmachung des Hinweises auf die Patenterteilung: **03.05.2006**
(21) Anmeldenummer: 03732372.2
(22) Anmeldetag: 14.05.2003
(51) Int. Cl.: A61B 17/74, A61B 17/72

(54) **OSTEOSYNTHESEEINRICHTUNG**
OSTEOSYNTHESIS DEVICE
DISPOSITIF D'OSTEOSYNTHESE

(30) Priorität: 14.05.2002 DE 20207733 U
(43) Veröffentlichungstag der Anmeldung: 16.02.2005
(73) Patentinhaber: Tantum AG, 24534 Neumünster (DE)
(72) Erfinder: JENSEN, Harm-Iven, 24214 Noer (DE); SCHOLZ, Hans-Joachim, 68199 Mannheim (DE); NIEMAX, Heinrich, 24534 Neumünster (DE)
(74) Vertreter: Wenzel & Kalkoff
(86) Internationale Anmeldenummer: PCT/EP2003/005060
(87) Internationale Veröffentlichungsnummer: WO 2003/094763

(56) Entgegenhaltungen:
- EP-A- 0 321 170
- EP-A- 0 838 199
- EP-A- 1 175 872
- GB-A- 2 209 947
- PATENT ABSTRACTS OF JAPAN vol. 1997, no. 04, 30. April 1997 (1997-04-30) & JP 08 322848 A (NARUSHIMA MASATO), 10. Dezember 1996 (1996-12-10)

## Beschreibung

Die Erfindung betrifft eine Osteosyntheseeinrichtung zur Versorgung trochantärer und subtrochantärer Femur-Frakturen, umfassend einen Femurnagel, der ein proximal in den Markraum des Femurs einführbarer Verriegelungsnagel mit wenigstens einer Querdurchbohrung zur Aufnahme einer Knochenschraube ist, eine Schenkelhalsschraube, die in einer Querdurchbohrung des Verriegelungsnagels axial verschiebbar geführt und mit ihrem proximalen Ende in den Schenkelhals des Femurs einführbar ist, und eine Fixiereinrichtung zur Verbindung der Schenkelhalsschraube mit dem Verriegelungsnagel, wobei die Fixiereinrichtung eine in dem proximalen Ende des Verriegelungsnagels vorgesehene Bohrung sowie ein in dieser angeordnetes Arretierelement aufweist, das zum Einstellen einer lösbaren Fixierposition der Schenkelhalsschraube mit einem dieser zugeordneten Abschnitt in Eingriff bringbar ist, wobei das Arretierelement in seiner Fixierposition eine axiale Bewegung der Schenkelhalsschraube zuläßt.

Üblicherweise weist der Verriegelungsnagel im distalen Bereich wenigstens eine Querbohrung für eine Knochenschraube auf, um ihn axial und in Drehrichtung festzulegen. Die im proximalen Bereich des Verriegelungsnagels vorgesehene Schenkelhalsschraube bringt den Kopf des Femurs in engen Eingriff mit dem Halsbereich des Femurknochens. Nach Reposition und bei Knochenheilung findet zumeist eine sogenannte Sinterung statt, die insbesondere durch erhebliche Belastungs- oder Muskelkräfte am Hüftgelenk bewirkt sein kann. Der Knochen kann sich im Bereich der Fraktur um einen erheblichen Sinterweg, der etliche Millimeter betragen kann, verkürzen. Die Schenkelhalsschraube muß der Verkürzung mit axialer Bewegung nachgeben, da andernfalls die Gefahr besteht, daß die Frakturversorgung instabil wird und sogar ein sogenannter Cut Out entstehen kann, indem die Schenkelhalsschraube den Femurkopf durchstößt.

Eine gattungsgemäße Osteosyntheseeinrichtung, die z.B. aus DE-U 87 01 164, DE-U 86 20 399 oder EP 0 321 170 (Basis für den Oberbegriff des Anspruchs 1) bekannt ist, weist eine Fixiereinrichtung auf, die einerseits die Schenkelhalsschraube gegen Drehung um ihre Achse durch Verriegelung sichert und andererseits eine freie axiale Verschiebbarkeit der Schenkelhalsschraube zuläßt.

Die Schenkelhalsschraube umfaßt eine axial sich erstreckende Nut, in die eine Blockierschraube als Verrieglungselement eingreift. Diese muß hart ausgeführt sein, um die Drehsicherung gegen Anschlag zu gewährleisten. Zudem muß die Blockierschraube von dem Nutboden freigehalten werden, um eine totale Arretierung der Schenkelhalsschraube in Axialrichtung zuverlässig zu vermeiden. Durch die freie axiale Verschiebbarkeit besteht die Gefahr, daß die Schenkelhalsschraube zum Beispiel bei der Versorgung osteoporotischer Knochen vollständig aus dem Verriegelungsnagel herauswandert. Andererseits wird die Blockierschraube im Bedarfsfall, wenn mittels der Schenkelhalsschraube die Knochen der Fraktur unter Zugspannung zusammenzuhalten sind, so stark angezogen, daß sie die Schenkelhalsschraube total arretiert, um sie unverschiebbar fest in ihrer Lage zu halten. In dieser Position der Blockierschraube kann die Schenkelhalsschraube einer Knochenverkürzung im Bereich der Fraktur nicht nachgeben, und es entsteht das Cut Out-Risiko.

Bei der aus EP 0 321 170 bekannten Einrichtung bleibt die Einstellung bzw. Bedienung der Blockier-Stellschraube unbefriedigend, da sichergestellt werden muß, daß sie in die Nut der Schenkelhalsschraube eingreift. Auch wird eine Justiereinstellung unter Verwendung einer dem Schaft der Schenkelhalsschraube entsprechenden Justierstange vorgeschlagen. Ferner werden Anschläge vorgeschlagen, um die Eingriffslänge der Blokkierschraube in die Nut zu bestimmen; zum vollständigen Blockieren, mit dem das Cut Out-Risiko verbunden ist, ist dann eine zweite Stellschraube erforderlich. In der Praxis der bekannten Osteosyntheseeinrichtung wird die freie axiale Verschiebbarkeit der Schenkelhalsschraube ein- und/oder beidseitig durch mechanische Anschläge begrenzt. Einerseits wird ein infolge der freigestellten Stellschraube relativ leichtes, mit Schmerz verbundenes Auswandern der Schenkelhalsschraube weiter nach außen begrenzt. Andererseits wird mittels einer zusätzlichen Schrägflächenbegrenzung ein Verschiebeweg an einem Teilweg der Schenkelhalsschraube ausgeschlossen, während sie unter Körpergewicht oder Muskeltätigkeit frei bewegbar bleiben soll. Man erkennt, daß die bekannte Osteosyntheseeinrichtung eine Reihe von Maßnahmen und besonderen Gestaltungen, die teilweise sogar mit einander entgegenwirkenden Funktionen verbunden sind, erfordert, um den Anforderungen der Femur-Frakturversorgung Rechnung tragen zu können.

Aus EP 1 175 872 ist ein intramedullärer Marknagel mit Schenkelhalsschraube und parallelem Schenkelhalsstift bekannt. Um die Schenkelhalsschraube gegen jede Bewegung gesichert mit dem Nagel zu verriegeln, wird sie mittels einer Verschlußschraube und eines von dieser mit Klemmkraft beaufschlagten, den Schenkelhalsstift hindurchlassenden Abstandshalters festgesetzt. Um auch den Schenkelhalsstift mit dem Nagel zu verriegeln, weist die Verschlußschraube zusätzlich einen gegen den Schenkelhalsstift zu spannenden Anschlag auf. Die Verriegelung beider Stabelemente mit dem Nagel mittels ein und derselben Verschlußschraube in Verbindung mit dem Abstandshalter erfordert infolge von Klemmkraft-Wechselwirkung zwischen den Teilen relativ hohe Spannkraft sowie besonders kritische Dimensionsanpassung der Teile aneinander. Relative axiale Bewegung zwischen Schenkelhalsschraube und Stift würde Instabilität und sogar Cut Out zur Folge haben.

Weiter sind unter dem Namen SYNTHES® Femurnägel bekannt, die Schenkelhalsschrauben aufnehmen, die jeweils eine mit flacher Schneidspitze endende Spiralklinge aufweisen. Die Schenkelhalsschraube weist über ihre Länge sich schraubenförmig windende, radial hervorspringende Klingenkanten auf. Um die Schenkelhalsschraube gegen jede Bewegung zu blockieren, werden Verschlußschrauben mit Polyethylenspitze verwendet, die derart deformierbar sind, daß sich eine Klingenkante beim Festziehen der Verschlußschraube profilartig in die Schraubenspitze hineindrückt, so daß die Schraubenspitze die Schneidkante schlitzartig aufnimmt. Zudem dienen die Verschlußschrauben zum statischen Verriegeln von die Schenkelhalsschrauben aufnehmenden Verriegelungshülsen mit den Femurnägeln. Um die Position der flachen Schneidspitze der Spiralklinge im Femurkopf zu sichern, muß jede Bewegung zuverlässig ausgeschlossen sein.

Der Erfindung liegt die Aufgabe zugrunde, eine Osteosyntheseeinrichtung zur Verbesserung der Versorgung trochantärer und subtrochantärer Frakturen zu schaffen, wobei insbesondere bei Knochensinterung das Cut Out-Risiko minimiert und/oder ein schmerzverursachendes laterales Herauswandem der Schenkelhalsschraube vermieden werden soll. Auch sollen operative Maßnahmen vereinfacht sein. Die Einrichtung soll einfach bauen und kostengünstig herstellbar sein.

Die Ziele der Erfindung werden in Verbindung mit den Merkmalen der eingangs genannten Osteosyntheseeinrichtung dadurch erreicht, daß das Arretierelement als Gleithemmungselement mit einem unter Fixier-Spannkraft wenigstens teilweise elastisch verformbaren Fixierkörper ausgebildet ist, der im die Fixierposition bestimmenden elastisch verformten Zustand an einem der Schenkelhalsschraube zugeordneten Gleitabschnitt derart angreift, daß längs des Gleitabschnitts wenigstens die axiale Fixierung der Schenkelhalsschraube bewirkende Haltekraft erzeugt wird, die durch statische Haltekraft sowie, infolge Last, durch wenigstens in axiale Richtung gehemmte Gleitbewegung zulassende Reib-Haltekraft bestimmt ist. Mit der erfindungsgemäßen Fixiereinrichtung wird erreicht, daß die Schenkelhalsschraube zugleich mit statischer und dynamischer Haltkraft derart blockierungsfrei gehalten wird, daß eine gebremste Gleit-Verschiebbarkeit zugelassen wird, um insbesondere einer Knochenverkürzung durch Sinterung zu entsprechen und/oder unerwünscht leichtgängiges laterales Auswandern der Schenkelhalsschraube zu verhindern. Herkömmliche Anschlag- und Blockierelemente werden vermieden. Dadurch ist die Schenkelhalsschraube zudem relativ einfach ausführbar. Die Erfindung vermeidet gezielt eine totale Arretierung und sieht statt dessen mittels des Arretier-Gleithemmungselements eine in Fixierposition die Schenkelhalsschraube zuverlässig festhaltende und kontinuierliche Einspannung vor, die gegen Gleitwiderstand eine dynamische Anpassung der Position der Schenkelhalsschraube an veränderte Zustände der Fraktur gewährleistet. Man erreicht, daß sich die Schenkelhalsschraube durch Mikrobewegung nicht selbsttätig lösen kann. Das Material des Arretierelements ist gummiartig elastisch. Selbst bei Kippbeanspruchung der Schenkelhalsschraube bleibt diese in ausreichendem Schluß mit dem Arretierelement verbunden. Haltekraft und Hemmkraft korrespondieren miteinander; letztere bremst die Gleitbewegung der Schenkelhalsschraube bei Überschreiten statischer Haltekraft. Im Ganzen wird eine Osteosyntheseeinrichtung erzielt, die einfache, operative Bedingungen verbessernde Anwendung sowie den Einsatz für sehr unterschiedliche Femur-Frakturstrukturen erlaubt.

Es ist gefunden worden, daß das erfindungsgemäß vorgesehene Arretierelement eine besonders vorteilhafte Charakteristik aufweist, wenn wenigstens das auf den Gleitabschnitt einwirkende Ende aus elastischem Material, vorzugsweise aus einem Silikonmaterial besteht. In bevorzugter Gestaltung ist das auf den Gleitabschnitt einwirkende Ende des Arretier-Gleithemmungselements derart federelastisch verformbar, daß es im Zustand der Fixierposition in Anschmiegung an eine Fläche des Gleitabschnittes eine entsprechend geformte Widerstandsgleitfläche ausbildet, wobei die Elastizität derart ausgeprägt ist, daß das Ende des Gleithemmungselements in freiem Zustand wenigstens nahezu seine ursprüngliche Form annimmt Zweckmäßig kann das auf den Gleitabschnitt einwirkende Ende des Arretier-Gleithemmungselements in unverformtem Zustand durch eine konvexe Fläche gebildet werden.

In besonderer Ausgestaltung der Erfindung ist die Schenkelhalsschraube drehbeweglich um ihre Achse gelagert, wobei das Arretier-Gleithemmungselement in seiner Fixierposition sowohl in axiale Richtung als auch in Drehrichtung gerichtete Haltekraft bzw. gleithemmende Reibkraft auf die Schenkelhalsschraube ausübt. Vorzugsweise ist der Gleitabschnitt durch einen Abschnitt der an der Innenwand der zugehörigen Querdurchbohrung geführten Mantelfläche der Schenkelhalsschraube gebildet. Haltekraft bzw. Gleit-Hemmkraft werden, vorzugsweise gleichmäßig, sowohl in Axialrichtung als auch in Drehrichtung der Schenkelhalsschraube kontinuierlich aufgebracht, wobei beide Bewegungsfreiheitsgrade zuverlässig von Blockierung bzw. Bewegungsstop durch Verriegelung befreit sind.

Eine andere vorteilhafte Ausgestaltung der Erfindung besteht darin, daß die Schenkelhalsschraube drehbeweglich um ihre Achse gelagert ist und das Arretier-Gleithemmungselement in seiner Fixierposition axiale Haltekraft bzw. axiale gleithemmende Reibkraft an dem der Schenkelhalsschraube zugeordneten Gleitabschnitt erzeugt, während die rotatorische Bewegbarkeit der Schenkelhalsschraube zumindest im wesentlichen unbeeinflußt bleibt. Mit diesen Maßnahmen erreicht man, daß sich der Kopf bzw. der Hals des Femur gegenüber dem den Verriegelungsnagel aufnehmenden Femurknochen nicht um die Achse der Schenkelhalsschraube drehen kann, so daß insoweit eine Drehsicherung zwischen den Knochenteilen hergestellt ist Andererseits bleibt die Schenkelhalsschraube in ihrer Lagerung an dem Verriegelungsnagel frei um ihre Achse drehbar, und zudem ist sie mittels des Arretier-Gleithemmungselements in axiale Richtung blockierungsfrei festgehalten sowie gegen Reibungs-Hemmkraft gleitverschiebbar. Dabei besteht eine bevorzugte Gestaltung der Erfindung darin, daß der der Schenkelhalsschraube zugeordnete Gleitabschnitt durch einen Schenkelhalsstift gebildet ist, an dessen distalem Ende die Schenkelhalsschraube um ihre Achse drehbar gelagert ist, wobei der Schenkelhalsstift in einer Querdurchbohrung des Verriegelungsnagels in gemeinsamer Verschiebbarkeit mit der Schenkelhalsschraube verschiebbar geführt und mit seinem proximalen Ende in den Femur-Schenkelhals einführbar ist.

In besonderer, ebenfalls bevorzugter Ausgestaltung umfaßt die Fixiereinrichtung ein das Arretier-Gleithemmungselement bildendes Stiftelement und eine diesem zugeordnete Fixierschraube, mittels der auf das Stiftelement in Richtung auf den Gleitabschnitt eines in den Femur-Schenkelhals einführbaren Stabkörpers Haltekraft bzw. gleithemmende Reibkraft erzeugende Fixierkraft ausübbar ist. Vorzugsweise können die Fixierschraube und das Stiftelement in unverlierbarer Verbindung, zweckmäßig in Steck-Preßverbindung, eine Funktions- und Handhabungseinheit bilden. Solche Schrauben/Stift-Arretiereinheiten lassen sich zur Bereitstellung steril verpacken, sie sind operativ besonders einfach und damit zuverlässig handhabbar, und deformierbare Stiftelemente lassen sich in verschiedenen Längen ausführen, um bei gleicher Fixierlänge unterschiedliche Halte- bzw. Gleithemmkraft definiert zu erzeugen. Im Zusammenhang mit dieser Ausgestaltung sieht die Erfindung vorzugsweise vor, daß das Arretier-Gleithemmungselement ein in seiner Axialrichtung definiert nachgiebiges Element ist, dem ein es in seiner Fixierposition aufnehmender Fixier-Bohrungsabschnitt vorgegebener Dimension zugeordnet ist. Im Rahmen dieser Ausgestaltung besteht eine bevorzugte Ausführung darin, daß in der Bohrung ein die Fixier-Bohrungslänge als vorgegebene Fixierlänge bestimmender Gewindeanschlag für ein mit dem Arretier-Gleithemmungselement zusammenwirkendes Gewinde, zweckmäßig einer Fixierschraube, ausgebildet ist.

Eine besonders zweckmäßige Ausgestaltung besteht darin, daß das Arretier-Gleithemmungselement in der Bohrung der Fixiereinrichtung geführt gehalten ist. In Verbindung mit einer solchen Führung kann der Fixierkörper vorzugsweise eine Textur aufweisen, die ihn im wesentlichen in Richtung parallel zur Führungsfläche Nachgiebigkeit zum Erzeugen der Halte-/Hemmkraft verleiht Die Fixierbohrung bildet so auch eine Abstützung für das Arretier-Gleithemmungselement.

Eine weitere Ausgestaltung des Arretier-Gleithemmungselements besteht darin, daß es, gegebenenfalls zusätzlich zu plastischer Verformbarkeit, in seiner axialen Richtung federelastisch nachgiebig ausgebildet ist.

Vorzugsweise ist das auf den Gleitabschnitt einwirkende Ende des Arretier-Gleithemmungselements in unverformtem Zustand durch eine konvexe Fläche gebildet. Infolge der elastischen Verformung im Bereich der Spitze vergrößert sich die Halte-/Hemmfläche des Fixierkörpers, wodurch Haltekraft und Gleithemmung erhöht werden.

In bevorzugter Erfindungsausführung ist die proximale Endseite des Verriegelungsnagels durch ein dem Arretier-Gleithemmungselement zugeordnetes Element, vorzugsweise durch eine Fixierschraube, verschlossen. Zweckmäßig schließt diese mit der proximalen Endseite' bündig ab. So wird sichergestellt, daß keine Substanzen in die Bohrung des Verriegelungsnagels bzw. in dessen Innengewinde am Nagelende wachsen können.

Unteransprüche sind auf die genannten und noch andere zweckmäßige und vorteilhafte Ausgestaltungen der Erfindung gerichtet. Besonders zweckmäßige und vorteilhafte Ausbildungsformen oder -möglichkeiten der Erfindung werden anhand der folgenden Beschreibung der in der schematischen Zeichnung dargestellten Ausführungsbeispiele beschrieben. Es zeigen
- Fig. 1: im Schnitt eine erfindungsgemäße Osteosyntheseeinrichtung mit in Axial- und Drehrichtung gehaltener und dabei gegen Hemmkraft gleitbewegbarer Schenkelhalsschraube,
- Fig. 2: im Längsschnitt eine erfindungsgemäße Osteosyntheseeinrichtung mit in Drehrichtung frei bewegbar gehaltener und in Achsrichtung fixierter sowie gegen Hemmkraft gleitverschiebbarer Schenkelhalsschraube und
- Fig. 3: im Schnitt ein Ausführungsbeispiel einer Fixierschraube und Gleithemmungselement umfassenden Einheit.

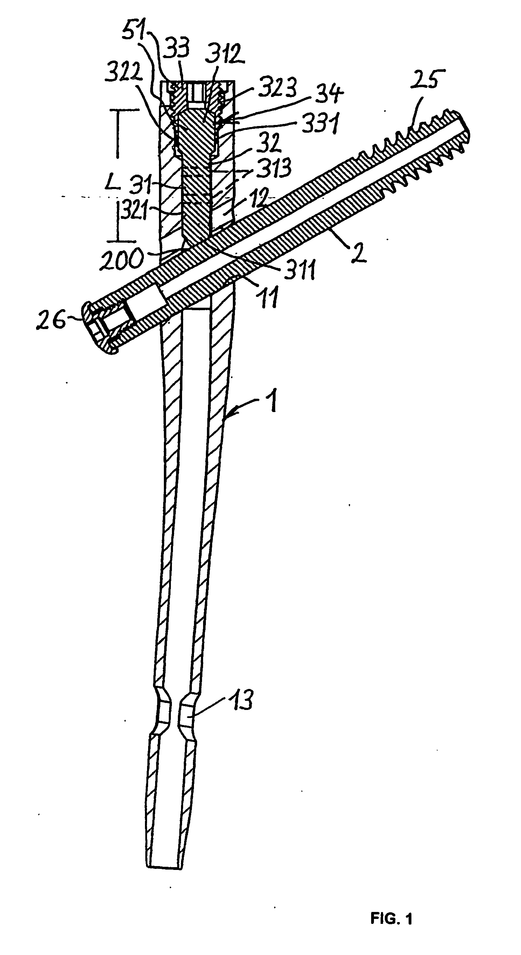

Die in Fig. 1 dargestellte Osteosyntheseeinrichtung umfaßt einen proximal in den Markraum des Femurs einführbaren Verriegelungsnagel 1, eine Schenkelhalsschraube 2 und eine Fixiereinrichtung 3 zum Verbinden der Schenkelhalsschraube 2 mit dem Verriegelungsnagel 1.

Am Ende des oberen Viertels des Verriegelungsnagels 1 ist eine schräge Querdurchbohrung-1 ausgebildet, deren Schräglage dem-CCD-Winkel und damit der Femur-Schenkelhalsneigung entspricht. Die Schenkelhalsschraube 2 weist einen kreiszylindrischen Abschnitt auf, dessen gekrümmte stetige Mantelfläche einen gleitfähigen Gleitabschnitt 200 bildet. Der zylindrische Abschnitt sitzt passend in der Querdurchbohrung 11, so daß die Schenkelhalsschraube 2 mittels der Bohrung 11 sowohl um ihre Achse drehbar als auch in Achsrichtung verschiebbar gelagert und geführt ist. Die Schenkelhalsschraube ist an ihrem proximalen Ende mit einem selbstschneidenden Gewinde 25 versehen, mit dem sie in den Femur-Schenkelhals einschraubbar ist. Das laterale Ende der Schenkelhalsschraube 2 ist mit einer Stirnschraube 26 abgeschlossen.

Der Verriegelungsnagel 1 weist kreiszylindrischen Querschnitt auf, der im proximalen oberen Viertel relativ groß ist und zum distalen Ende des Nagels 1 hin abnimmt. In dem unteren Bereich des Verriegelungsnagels 1 ist eine Querdurchbohrung 13 eingebracht. Durch diese wird eine Knochenschraube hindurchgesetzt, um den Verriegelungsnagel 1 nach dem Einführen in das Femur axial und in Drehrichtung festzulegen.

Das proximale obere Viertel des Verriegelungsnagels 1 weist eine abgestufte Bohrung 32 mit einem aufgeweiteten oberen Abschnitt 322 und einem unteren Abschnitt 321 geringeren Durchmessers auf. Die am proximalen Ende des Verriegelungsnagels 1 offene Bohrung 32 öffnet sich in die Querdurchbohrung 11 hinein. Die Bohrung 32 ist Bestandteil der Fixiereinrichtung 3. Diese umfaßt ein Stiftelement 31 und eine Fixierschraube 33, mittels der auf das Stiftelement 31 in Richtung auf den Gleitabschnitt 200 der Schenkelhalsschraube 2, die einen Stabkörper bildet, eine Fixier-Spannkraft ausübbar ist, die an dem Gleitabschnitt 200 sowohl statischen Haltekraft als auch gleithemmende Halte-Reibkraft erzeugt. Mittels statischer und dynamischer Haltekraft wird die Schenkelhalsschraube 2 in Drehrichtung und Axialrichtung gleichermaßen fixiert, wobei sie, infolge Last, bei Überschreiten der statischen Haltekraft gleichermaßen einerseits in Achsrichtung und andererseits in Drehrichtung gleit-verschiebbar ist Nach Maßgabe der Fixier-Spannkzaft wird die Gleitbewegung gehemmt, so daß die Schenkelhalsschraube 2 bei Last Bewegungen mit den beiden Richtungs-Freiheitsgraden zwar blockierungsfrei folgen kann, dennoch aber der sichere selbsttätige Halt der Schenkelhalsschraube 2 gewährleistet ist.

Man erkennt, daß das Stiftelement 31 ein Arretier-Gleithemmungselement in Form eines Fixierkörpers ist, der bei Beaufschlagung mit Spannkraft Halte- bzw. Gleithemmkraft an dem Gleitabschnitt 200 erzeugt. Das Stiftelement 31 sitzt mit einem unteren Abschnitt passend in dem unteren, den Durchmesser Q aufweisenden Abschnitt 321 der Bohrung 32, so daß das Stiftelement 31 dort geführt bzw. abgestützt gehalten ist. Das freie Ende des Stiftelements 31 ist als Stiftfuß 311 ausgebildet, der in unverformtem Zustand gerundet mit konvex gewölbter oder kegelartiger Fläche oder Spitze ausgebildet ist. Der Fuß 311 des Fixierkörpers ist unter Wirkung der Spannkraft elastisch verformbar und damit derart nachgiebig, daß an dem Gleitabschnitt 200 eine ausgeprägte form- und bedingt kraftschlüssige Auflage- und Anlagefläche gebildet wird, die die Halte- bzw. Gleithemmkraft bewirkt. Zwar ist es auch denkbar, daß der Stiftfuß plastisch verformbar ist. Das Stiftelement 31 wird dann im Bereich oberhalb des Fußes 311 elastisch nachgiebig ausgebildet sein. Zweckmäßig können zwei gestrichelt dargestellte umlaufende nutartige Einschnitte oder Einstiche 313 ausgebildet werden, mittels derer das Stiftelement 31 im wesentlichen in Richtung seiner Achse federelastisch nachgiebig ist. Um dem Stiftelement die erforderlichen elastischen und ggf. zusätzlich plastischen Charakteristika zu verleihen, besteht es aus geeignet eingestelltem Kunststoffmaterial. Es ist gefunden worden, daß Silikonmaterialien besonders geeignet sind. Es werden solche Materialien bzw. Silikone gewählt, die derart elastisch verformbar sind, daß der Stiftfuß 311 im Zustand seiner Fixierposition in backenartiger Anschmiegung an die stetige Fläche des Gleitabschnitts 200 eine entsprechend geformte stetige Widerstandsgleitfläche ausbildet. Die Elastizität des Materials wird derart eingestellt bzw. ausgeprägt gewählt, daß der Stiftfuß 311 in freiem Zustand wenigstens nahezu seine ursprüngliche Form annimmt. Die Elastizitätseigenschaft des Materials ist zudem derart, daß in elastisch deformiertem Zustand eine ausreichend feste, homogene und stetige, sich an die konvexe Fläche des Gleitabschnitts 200 anschmiegende Haltefläche bzw. Widerstands-Gleitfläche für kontrolliert gebremste Gleitbewegung gebildet wird. Die Haftung zwischen dem Stiftfuß 311 und der Schenkelhalsschraube 2 bzw. dem Gleitabschnitt 200 bleibt auch dann optimal, wenn bei axialer Belastung der Schraube 2 Kippmomente entstehen. Lockerung und Instabilität werden dadurch zuverlässig ausgeschlossen. Mit der Wahl eines geeigneten Gummi- bzw. Silikonmaterials wird auch sichergestellt, daß die gehemmte Gleitbewegung stetig und blockierungsfrei bleibt. Insbesondere ist gefunden worden, daß Polyethylenmaterialien mangels ausreichender Elastizität für die erfindungsgemäßen Stiftelemente 31 jedenfalls ohne weiteres nicht geeignet sind.

Das Stiftelement 31 weist einen Stiftkopf 312 mit gegenüber dem in dem unteren Bohrungsabschnitt 321 abgestützten Schaftteil größerem Durchmesserquerschnitt auf. Der Kopf 312 ragt frei in den oberen Bohrungsabschnitt 322. Die Fixierschraube 33 weist eine Steckbuchse auf, mit der sie auf den Kopf 312 im Stecksitz aufgepreßt ist. Die Fixierschraube 33 und das Stiftelement 31 bilden eine Funktions- und Handhabungseinheit 5, die sich als Element mit unverlierbaren Teilen bequem und zuverlässig in die Axialbohrung 32 des Verriegelungsnagels 1 einsetzen bzw. von dort entnehmen läßt.

Der obere Bohrungsabschnitt 322 ist mit einem Gewindeabschnitt 323 versehen, in den das Gewinde der als Gewindestopfen ausgeführten Fixierschraube 33 eingreift. Die Fixierschraube 33 weist einen Hohlkopf auf, der in versenkter Anordnung im eingeschraubten Zustand bündig mit der Endfläche des Verriegelungsnagels 1 abschließt. In dieser Position ist die Fixierschraube 33 gegen einen Gewindeanschlag 331 an ihrem dem Stiftkopf 312 zugewandten Ende des Gewindeabschnitts 323 gesetzt. Dadurch wird zwischen dem Boden der Steckbuchse der Fixierschraube 33 und der Gleit-Mantelfläche 200 der Schenkelhalsschraube 2 eine konstante axiale Fixier-Bohrungslänge L bestimmt, die kleiner als die entsprechende Länge des unverformten Stiftelements 31 ist. Durch die Dimensionen Q und L wird eine Art Klemmraum für das Stiftelement 31 bestimmt, wobei das Stiftelement 31 kein blockierendes, sondern ein hemmende Gleitreibung sicherstellendes Klemmelement ist.

Einerseits ist erreicht, daß gleiche aus Fixierschraube 33 und Stiftelement 31 zusammengefügte Einheiten 51 in eingeschraubtem, gegen den Gewindeanschlag 331 gesetztem Zustand jeweils die gleiche Halte- bzw. Gleit-Hemmkraft bei Verwendung gleicher Schenkelhalsschrauben 2 erzeugen. Andererseits können gleiche Fixierschrauben 33 verschieden lange Stiftelemente 31 aufnehmen. Die verschiedenen Einheiten 31, 33 erzeugen dann entsprechend verschiedene Kräfte, die durch die Stiftlängen und die federelastischen Stift-Charakteristika bestimmt sind, wobei die Halte- bzw. Gleit-Hemmverbindung in jedem Falle durch einfache Verwendung der gewählten Einheit 5 zuverlässig vorgebbar und reproduzierbar ist.

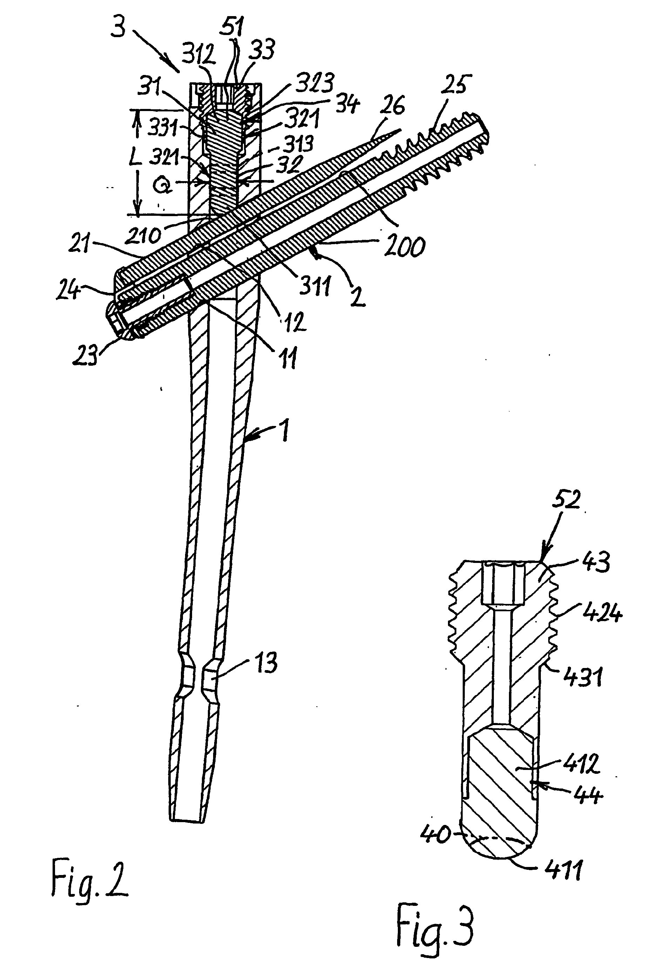

Die Osteosyntheseeinrichtung gemäß Fig. 2 weist eine Schenkelhalsschraube 2 auf, die mit einem Schenkelhalsstift 21 verbunden ist. Verriegelungsnagel 1 und Fixiereinrichtung 3 sind wie in Fig. 1 ausgebildet, und für entsprechende Teile werden gleiche Bezugszeichen verwendet. Der Unterschied der Ausführungsform gemäß Fig. 2 besteht darin, daß die Schenkelhalsschraube 2 frei drehbar um ihre Achse gelagert ist, während sie in Achsrichtung gegen Haltekraft bzw. Gleithemmkraft gleitverschiebbar fixiert und geführt ist.

Der Verriegelungsnagel 1 weist zu der Querdurchbohrung 11 eine Neben-Querdurchbohrung 12 auf, deren Durchmesser geringer als der der Bohrung 11 ist und die den einen stabartigen Körper bildenden Schenkelhalsstift 21 passend aufnimmt und führt. Das proximale Ende 26 des Schenkelhalsstifts ist als Spitze ausgeformt, um ihn einfach in den Femurhals eintreiben zu können.

Die Bohrung 12 erstreckt sich auf der dem proximalen Ende des Nagels 1 zugewandten Seite der Schenkelhalsschraube 2 parallel mit und in geringem Abstand zu der Bohrung 11. Die Schenkelhalsschraube 2 ist an dem distalen Ende des Schenkelhalsstifts 21 frei drehbar gelagert. Diese Lagerung umfaßt einen Steg 24, mit dem einerseits die Schenkelhalsschraube 2 mittels einer Lagerschraube 23 drehbar verbunden ist und mit dem andererseits der Schenkelhalsstift 21 starr verbunden ist. Dadurch ist erreicht, daß die Schenkelhalsschraube 2 und der Schenkelhalsstift 21 in Form einer Verschiebeeinheit gemeinsam in zur Achse der Schenkelhalsschraube 2 paralleler Richtung verschiebbar und gleitbewegbar geführt sind. Die Oberfläche des Schenkelhalsstifts 21 ist als Gleitfläche ausgebildet. Im Bedarfsfall kann es aber auch zweckmäßig sein, die Schenkelhalsschraube 2 lateralseitig derart fest mit dem distalen Ende des Schenkelhalsstifts 21 zu verbinden, daß die Schenkelhalsschraube 2 gegen Rotation um ihre Achse gesichert wird.

Das Stiftelement 31 ist kürzer als im Ausführungsbeispiel der Fig. 1 ausgeführt und greift an der dem proximalen Ende zugewandten Seite des Schenkelhalsstiftes 21 an dem Gleitabschnitt 210 der Gleitoberfläche des Schenkelhalsstiftes 21 an. Man erkennt, daß die Axialposition der aus Schenkelhalsstift 21 und Schenkelhalsschraube 2 bestehenden Verschiebeeinheit nun durch die auf den Schenkelhalsstift 21 wirkende Kraft am Fuß des Stiftelements 31 statisch und zugleich dynamisch gegen bremsende Gleit-Hemmkraft fixiert ist.

Der mit der Nebenbohrung 12 gemäß Fig. 1 und 2 versehene Verriegelungsnagel 1 kann wahlweise für die Schenkelhalsschraube 2 ohne bzw. mit Schenkelhalsstift 21 genutzt werden. Für die Nutzung mit Schenkelhalsstift 21 werden Arretierungseinheiten 31, 33 mit Stiftelementen 31 verwendet, deren Axiallängen auf den Abstand der Nebenbohrung 12 vom proximalen Ende des Verriegelungsnagels 1 bzw. von dem Boden der Steckbuchse der Fixierschraube angepaßt sind.

In Fig. 3 ist eine modifizierte Funktions- und Handhabungseinheit 52, im Preßsitz zusammengefügt aus einem ein Gleithemmungselement bildenden Stiftelement 41 und einer Fixierschraube 43, dargestellt Diese Einheit 52 wird anstelle der Einheit 51 in Fig. 1 und 2 verwendet Die Fixierschraube 43 ist gleichfalls als Stopfen mit Gewinde 424 ausgeführt. Im Unterschied zu den Ausführungsbeispielen der Fig. 1 und 2 ist die Fixierschraube 43 länger als die Fixierschraube 33 ausgeführt Sie weist eine Buchse auf, die so dimensioniert ist, daß sie einen Kopfabschnitt des Stiftelements 41 aufnimmt, der ausgeprägt länger als der an der Fixierschraube 43 hervorstehende Fußabschnitt des Stiftelements 41 ist. Dadurch wird das aus einem Silikon gebildete Stiftelement 41, dessen Kopfabschnitt in die Schraubenbuchse zweckmäßig in Spritzgußverbindung 44 eingepreßt worden ist, besonders optimal gestützt und gehalten, um am Stiftfuß 411 die durch das federelastische Material erzielten Funktionen zum Halten und zur Gleithemmung mit optimalem Deformationsbereich am Fußende 411 besonders wirksam hervorzubringen. Das Bezugszeichen 40 bezeichnet strich-punktiert die in der Fixierposition gebildete Halte- und Widerstandsgleitfläche, die durch elastische Materialverdrängung entsprechend der Kontur des Gleitabschnitts 200 bzw. 210 entsteht. Die Schraube 43 weist an der Kopfunterseite einen umlaufenden Schrägrand auf, der wie in den Ausführungsbeispielen der Fig. 1 und 2 einen Gewindeanschlag 431 bildet, um die Fixierlänge des Silikon-Stiftelements 41 definiert und reproduzierbar zu bestimmen.

## Patentansprüche

1. Osteosyntheseeinrichtung zur Versorgung trochantärer und subtrochantärer Femur-Frakturen, umfassend einen Femurnagel, der ein proximal in den Markraum des Femurs einführbarer Verriegelungsnagel (1) mit wenigstens einer Querdurchbohrung (11, 13) zur Aufnahme einer Knochenschraube (2) ist, eine Sehenkelhalsschraube (2), die in einer Querdurchbohrung (11) des Verriegelungsnagels (1) axial verschiebbar geführt und mit ihrem proximalen Ende in den Schenkelhals des Femurs einführbar ist, und eine Fixiereinrichtung (3) zur Verbindung der Schenkelhalsschraube (2) mit dem Verriegelungsnagel (1), wobei die Fixiereinrichtung (3) eine in dem proximalen Ende des Verriegelungsnagels (1) vorgesehene Bohrung (32) sowie ein in dieser angeordnetes Arretierelement (31, 41) aufweist, das zum Einstellen einer lösbaren Fixierposition der Schenkelhalsschraube (2) mit einem dieser zugeordneten Abschnitt (200, 210) in Eingriff bringbar ist, wobei das Arretierelement (31,41) in seiner Fixierposition eine axiale Bewegung der Schenkelhalsschraube (2) zuläßt, **dadurch ge- kennzeichnet,** daß das Arretierelement als Gleithemmungselement (31, 41) mit einem unter Fixier-Spannkraft wenigstens teilweise elastisch verformbaren Fixierkörper ausgebildet ist, der im die Fixierposition bestimmenden elastisch verformten Zustand an einem der Schenkelhalsschraube (2) zugeordneten Gleitabschnitt (200, 210) derart angreift, daß längs des Gleitabschnitts (200, 210) wenigstens die axiale Fixierung der Schenkelhalsschraube (2) bewirkende Haltekraft erzeugt wird, die durch statische Haltekraft sowie, infolge Last, durch wenigstens in axiale Richtung gehemmte Gleitbewegung zulassende Reib-Haltekraft bestimmt ist.

2. Einrichtung nach Anspruch 1, **dadurch gekennzeichnet, daß** wenigstens das auf den Gleitabschnitt (200, 210) einwirkende Ende (311) des Gleithemmungselements (31) aus elastischem Material, vorzugsweise aus einem Silikon-Material besteht.

3. Einrichtung nach Anspruch 1 oder 2, **dadurch gekennzeichnet, daß** das auf den Gleitabschnitt (200, 210) einwirkende Ende (311, 411) des Arretier-Gleithemmungselements (31, 41) derart federelastisch verformbar ist, daß es im Zustand der Fixierposition in Anschmiegung an eine Fläche des Gleitabschnittes (200, 210) eine entsprechend geformte Widerstandsgleitfläche (40) ausbildet, wobei die Elastizität derart ausgeprägt ist, daß das Ende (311, 411) des Gleithemmungselements (31, 41) in freiem Zustand wenigstens nahezu seine ursprüngliche Form annimmt.

4. Einrichtung nach einem der Ansprüche 1 bis 3, **dadurch gekenn- zeichnet**, daß das auf den Gleitabschnitt (200, 210) einwirkende Ende (311) des Arretier-Gleithemmungselements (31, 41) in unverformtem Zustand durch eine konvexe Fläche gebildet ist.

5. Einrichtung nach einem der Ansprüche 1 bis 4, **dadurch gekenn- zeichnet,** daß sich die Bohrung (32) der Fixiereinrichtung (3) von ihrer am proximalen Ende des Verriegelungsnagels (1) offenen Seite bis in die Querdurchbohrung (11, 12) erstreckt, die einen in den Femur-Schenkelhals einführbaren stabartigen Körper (2, 21) führt, der den mit dem Arretier-Gleithemmungselement (31, 41) zusammenwirkenden Gleitabschnitt (200, 210) aufweist.

6. Einrichtung nach Anspruch 5, **dadurch gekennzeichnet, daß** die Fixiereinrichtung (3) ein das Arretier-Gleithemmungselement bildendes Stiftelement (31,41) und eine diesem zugeordnete Fixierschraube (33, 43) umfaßt, mittels der auf das Stiftelement (31, 41) in Richtung auf den Gleitabschnitt (200, 210) des in den Femur-Schenkelhals einführbaren Stabkörpers (2,21) Halte- bzw. gleithemmende Reibkraft erzeugende Fixierkraft ausübbar ist.

7. Einrichtung nach Anspruch 6, **dadurch gekennzeichnet, daß** die Fixierschraube (33, 43) und das Stiftelement (31, 41) in unverlierbarer Verbindung, vorzugsweise in Preßverbindung (34, 44), eine Funktions- und Handhabungseinheit (51, 52) bilden.

8. Einrichtung nach einem der Ansprüche 1 bis 7, **dadurch gekennzeichnet, daß** das Arretier-Gleithemmungselement (31, 41) in der Bohrung (32) der Fixiereinrichtung (3) geführt gehalten ist.

9. Einrichtung nach einem der Ansprüche 1 bis 8, **dadurch gekennzeichnet, daß** das Arretier-Gleithemmungselement (31, 41) in seiner axialen Richtung-federelastisch nachgiebig ausgebildet ist.

10. Einrichtung nach einem der Ansprüche 1 bis 9, **dadurch gekennzeichnet, daß** das Arretier-Gleithemmungselement (31, 41) ein in seiner Axialrichtung definiert elastisch nachgiebiges Element ist, dem ein es in seiner Fixierposition aufnehmender Fixier-Bohrungsabschnitt vorgegebener Dimension (L, Q) zugeordnet ist.

11. Einrichtung nach Anspruch 10, **dadurch gekennzeichnet, daß** in der Bohrung (32) ein Gewindeanschlag (321) für ein mit dem Arretier-Gleithemmungselement (31, 41) zusammenwirkendes Gewinde, vorzugsweise einer Fixierschraube (33, 43) ausgebildet ist, der eine Fixier-Bohrungslänge (L) als vorgegebene Dimension bestimmt.

12. Einrichtung nach einem der Ansprüche 1 bis 11, **dadurch gekennzeichnet, daß** die proximale Endseite des Verriegelungsnagels (2) durch ein dem Arretier-Gleithemmungselement (31, 41) zugeordnetes Element, vorzugsweise durch eine Fixierschraube (33, 43), verschlossen ist, die vorzugsweise mit der Endseite bündig abschließt.

13. Einrichtung nach einem der Ansprüche 1 bis 12, **dadurch gekennzeichnet, daß** die Schenkelhalsschraube (2) drehbeweglich um ihre Achse gelagert ist und das Arretier-Gleithemmungselement (31, 41) in seiner Fixierposition sowohl in axiale Richtung als auch in Drehrichtung gerichtete Haltekraft bzw. gleithemmende Reibkraft, vorzugsweise gleichermaßen, auf die Schenkelhalsschraube (2) ausübt, wobei vorzugsweise der Gleitabschnitt durch einen Abschnitt (200) der an der Innenwand der zugehörigen Querdurchbohrung (11) geführten Mantelfläche der Schenkelhalsschraube (2) gebildet ist.

14. Einrichtung nach einem der Ansprüche 1 bis 12, **dadurch gekennzeichnet, daß** die Schenkelhalsschraube (2) drehbeweglich um ihre Achse gelagert ist und das Arretier-Gleithemmungselement (31, 41) in seiner Fixierposition axiale Haltekraft bzw. axiale gleithemmende Reibkraft an dem der Schenkelhalsschraube (2) zugeordneten Gleitabschnitt (210) erzeugt, während die rotatorische Bewegbarkeit der Schenkelhalsschraube (2) zumindest im wesentlichen unbeeinflußt bleibt.

15. Einrichtung nach Anspruch 14, **dadurch gekennzeichnet, daß** der der Schenkelhalsschraube (2) zugeordnete Gleitabschnitt (210) durch einen Schenkelhalsstift (21) gebildet ist, an dessen distalem Ende die Schenkelhalsschraube (2) um ihre Achse drehbar gelagert ist, wobei der Schenkelhalsstift (21) in einer Querdurchbohrung (12) des Verriegelungsnagels (1) in gemeinsamer Verschiebbarkeit mit der Schenkelhalsschraube (2) verschiebbar geführt und mit seinem proximalen Ende (26) in den Femur-Schenkelhals einführbar ist.

16. Einrichtung nach einem der Ansprüche 1 bis 12, **dadurch gekennzeichnet, daß** der der Schenkelhalsschraube (2) zugeordnete Gleitabschnitt (210) durch einen Schenkelhalsstift (21) gebildet ist, mit dessen distalem Ende die Schenkelhalsschraube (2) gegen Rotation gesichert fest verbunden ist, wobei der Schenkelhalsstift (21) in einer Querdurchbohrung (12) des Verriegelungsnagels (1) in gemeinsamer Verschiebbarkeit mit der Schenkelhalsschraube (2) verschiebbar geführt und mit seinem proximalen Ende (26) in den Femur-Schenkelhals einführbar ist.

## Claims

1. Osteosynthesis device for the care of trochanteric and subtrochanteric femur fractures, including a femur nail which is a locking nail (1) with at least one transverse through-hole (11, 13) that can be introduced proximally into the marrow space of the femur, for receiving a bone screw (2), a femoral neck screw (2) which is guided axially slidably in a transverse through-hole (11) of the locking nail (1) and can be introduced by its proximal end into the neck of the femur, and a fixing device (3) for connecting the femoral neck screw (2) to the locking nail (1), wherein the fixing device (3) has a bore (32) provided in the proximal end of the locking nail (1) and a locking element (31, 41) arranged therein which, to adjust its releasable position of fixing the femoral neck screw (2), can be engaged with a section (200, 210) associated with the latter, wherein the locking element (31, 41) in its fixing position allows an axial movement of the femoral neck screw (2), **characterized in that** the locking element is designed as a slide-retarding element (31, 41) with a fixing body which is at least partially elastically deformable under fixing tension and which in the elastically deformed state defining the fixing position engages a sliding section (200, 210) associated with the femoral neck screw (2) in such a way that along the sliding section (200, 210) is generated at least a retaining force which causes axial fixing of the femoral neck screw (2) and which is determined by a static retaining force and, as a result of load, by a friction retaining force which allows at least sliding movement that is inhibited in the axial direction.

2. Device according to claim 1, **characterized in that** the end (311) of the slide-retarding element (31) acting on the sliding section (200, 210) is made of elastic material, preferably a silicone material.

3. Device according to claim 1 or 2, **characterized in that** the end (311, 411) of the locking/slide-retarding element (31, 41) acting on the sliding section (200, 210) is elastically deformable in such a way that in the state of the fixing position fitting snugly against a surface of the sliding section (200, 210) it forms a correspondingly shaped resistance sliding surface (40), wherein the elasticity is so pronounced that the end (311, 411) of the slide-retarding element (31, 41) in the free state at least nearly assumes its original shape.

4. Device according to any of claims 1 to 3, **characterized in that** the end (311) of the locking/slide-retarding element (31, 41) acting on the sliding section (200, 210) in the undeformed state is formed by a convex surface.

5. Device according to any of claims 1 to 4, **characterized in that** the bore (32) of the fixing device (3) extends from its open side at the proximal end of the locking nail (1) to the transverse through-hole (11, 12) which guides a rod-like body (2, 21) which can be introduced into the neck of the femur and which comprises the sliding section (200, 210) that cooperates with the locking/slide-retarding element (31, 41).

6. Device according to claim 5, **characterized in that** the fixing device (3) includes a pin element (31, 41) forming the locking/slide-retarding element, and a fixing screw (33, 43) which is associated with the pin element (31, 41) and by means of which a fixing force generating a retaining or slide-retarding friction force can be exerted on the pin element (31, 41) in a direction towards the sliding section (200, 210) of the rod body (2,21) that can be introduced into the neck of the femur.

7. Device according to claim 6, **characterized in that** the fixing screw (33, 43) and the pin element (31, 41) in a captive joint, preferably in a compression joint (34, 44), form an operating and handling unit (51, 52).

8. Device according to any of claims 1 to 7, **characterized in that** the slide-retarding locking element (31, 41) is held in guided fashion in the bore (32) of the fixing device (3).

9. Device according to any of claims 1 to 8, **characterized in that** the locking/slide-retarding element (31, 41) is elastically yielding in its axial direction.

10. Device according to any of claims 1 to 9, **characterized in that** the locking/slide-retarding element (31, 41) is an element with defined elastic yielding in its axial direction, with which is associated a fixing bore section of predetermined dimension (L, Q) which receives it in its fixing position.

11. Device according to claim 10, **characterized in that** in the bore (32) is formed a threaded stop (321) for a thread cooperating with the locking/slide-retarding element (31, 41), preferably a fixing screw (33, 43), which stop determines a fixing bore length (L) as a predetermined dimension.

12. Device according to any of claims 1 to 11, **characterized in that** the proximal end side of the locking nail (2) is closed by an element associated with the locking/slide-retarding element (31, 41), preferably by a fixing screw (33, 43) which preferably ends flush with the end side.

13. Device according to any of claims 1 to 12, **characterized in that** the femoral neck screw (2) is mounted rotatably about its axis, and the locking/slide-retarding element (31, 41) in its fixing position exerts a retaining force or slide-retarding friction force directed both in the axial direction and in the direction of rotation, preferably equally, on the femoral neck screw (2), wherein preferably the sliding section is formed by a section (200) of the peripheral surface of the femoral neck screw (2), which peripheral surface is guided on the inner wall of the associated transverse through-hole (11).

14. Device according to any of claims 1 to 12, **characterized in that** the femoral neck screw (2) is mounted rotatably about its axis, and the locking/slide-retarding element (31, 41) in its fixing position generates an axial retaining force or axial slide-retarding friction force on the sliding section (210) associated with the femoral neck screw (2), while the rotational mobility of the femoral neck screw (2) remains at least substantially unaffected.

15. Device according to claim 14, **characterized in that** the sliding section (210) associated with the femoral neck screw (2) is formed by a femoral neck pin (21) at the distal end of which the femoral neck screw (2) is mounted so as to be rotatable about its axis, wherein the femoral neck pin (21) is guided slidably in a transverse through-hole (12) in the locking nail (1) in joint slidability with the femoral neck screw (2) and can be introduced by its proximal end (26) into the neck of the femur.

16. Device according to any of claims 1 to 12, **characterized in that** the sliding section (210) associated with the femoral neck screw (2) is formed by a femoral neck pin (21) to the distal end of which the femoral neck screw (2) is rigidly connected so as to be prevented from rotating, wherein the femoral neck pin (21) is guided slidably in a transverse through-hole (12) in the locking nail (1) in joint slidability with the femoral neck screw (2) and can be introduced by its proximal end (26) into the neck of the femur.

## Revendications

1. Dispositif d'ostéosynthèse destiné à des fractures du fémur dans la région trochantérienne et sub-trochantérienne, lequel dispositif comporte un clou fémoral qui est un clou de verrouillage (1) insérable du côté proximal dans l'espace médullaire du fémur et comportant au moins un perçage transversal (11, 13) destiné à recevoir une vis pour os (2), une vis de col de fémur (2) qui est guidée axialement en translation dans un perçage transversal (11) du clou de verrouillage (1) et qui est insérable par son extrémité proximale dans le col du fémur, et un dispositif de fixation (3) destiné à relier la vis de col de fémur (2) au clou de verrouillage (1), le dispositif de fixation (3) comportant un perçage (32) ménagé dans l'extrémité proximale du clou de verrouillage (1) ainsi qu'un élément d'arrêt (31, 41) qui est placé dans ce perçage et qui peut être amené en engagement avec une portion (200, 210) de la vis de col de fémur (2) afin de régler la position de fixation mobile de cette vis de col de fémur, l'élément d'arrêt (31, 41) permettant dans sa position de fixation un mouvement axial de la vis de col de fémur (2), **caractérisé en ce que** l'élément d'arrêt est conformé en élément de freinage glissant (31, 41) comportant un corps de fixation qui est au moins partiellement déformable élastiquement sous l'action d'une force de serrage et de fixation et qui est appliqué sur une portion de glissement (200, 210) associée à la vis de col de fémur (2) dans l'état déformé élastiquement définissant la position de fixation de façon à générer le long de la portion de glissement (200, 210) une force de maintien qui provoque au moins la fixation axiale de la vis de col de fémur (2) et qui est définie à la suite d'un effort, par une force de maintien et de friction permettant un mouvement de glissement freiné au moins dans une direction axiale.

2. Dispositif selon la revendication 1, **caractérisé en ce qu'**au moins l'extrémité (311), agissant sur la portion de glissement (200, 210) de l'élément de freinage glissant (31) est en matériau élastique, avantageusement en silicone.

3. Dispositif selon la revendication 1 ou 2, **caractérisé en ce que** l'extrémité (311, 411), agissant sur la portion de glissement (200, 210) de l'élément d'arrêt/de freinage glissant (31, 41) est déformable élastiquement de manière à former, dans l'état de la position de fixation en appui contre une surface de la portion de glissement (200, 210), une surface de glissement antagoniste (40) de forme correspondante, l'élasticité étant telle que l'extrémité (311, 411) de l'élément à freinage glissant (31, 41) prend à l'état libre au moins approximativement sa forme d'origine.

4. Dispositif selon l'une des revendications 1 à 3, **caractérisé en ce que** l'extrémité (311), agissant sur la portion de glissement (200, 210), de l'élément d'arrêt /de freinage glissant (31, 41) est formée, dans un état déformé, par une surface convexe.

5. Dispositif selon l'une des revendications 1 à 4, **caractérisé en ce que** le perçage (32) du dispositif de fixation s'étend depuis son côté ouvert à l'extrémité proximale du clou de verrouillage (1) dans le perçage traversant transversal (11, 12) qui guide un corps (2, 21) en forme de barre qui peut être inséré dans le col de fémur et qui comporte la portion de glissement (200, 210) coopérant avec l'élément d'arrêt/de freinage glissant (31, 41).

6. Dispositif selon la revendication 5, **caractérisé en ce que** le dispositif de fixation (3) comporte un élément de type broche (31, 41) formant l'élément d'arrêt/de freinage glissant et une vis de fixation (33, 43) qui est associée à cet élément de type broche et au moyen de laquelle une force de fixation crée une force de maintien respectivement de friction à freinage glissant, sur l'élément de type broche (31, 41) en direction de la portion de glissement (200, 210) du corps (2, 21) en forme de barre qui peut être inséré dans le col de fémur.

7. Dispositif selon la revendication 6, **caractérisé en ce que** la vis de fixation (33, 43) et l'élément de type broche (31, 41) forme dans une liaison imperdable, avantageusement une liaison par pressage (34, 44), avec une unité fonctionnelle et opératoire (51, 52).

8. Dispositif selon l'une des revendications 1 à 7, **caractérisé en ce que** l'élément d'arrêt/de freinage glissant (31, 41) est maintenu en étant guidé dans le perçage (32) du dispositif de fixation (3).

9. Dispositif selon l'une des revendications 1 à 8, **caractérisé en ce que** l'élément d'arrêt/de freinage glissant (31, 41) est conformé pour être flexible élastiquement dans sa direction axiale.

10. Dispositif selon l'une des revendications 1 à 9, **caractérisé en ce que** l'élément d'arrêt/de freinage glissant (31, 41) est un élément qui est flexible élastiquement de façon définie dans sa direction axiale et auquel est associée une portion de perçage de fixation de dimension prédéterminée (L, Q) qui reçoit cet élément dans sa position de fixation.

11. Dispositif selon la revendication 10, **caractérisé en ce qu'**une butée filetée (321) destinée à un taraudage coopérant avec l'élément d'arrêt/de freinage glissant (31, 41), avantageusement une vis de fixation (33, 43), qui détermine une longueur de perçage (L) de fixation de dimension prédéterminée, est conformée dans le perçage (32).

12. Dispositif selon l'une des revendications 1 à 11, **caractérisé en ce que** l'extrémité proximale du clou de verrouillage (2) est fermée par un élément, associé à un élément d'arrêt/de freinage glissant (31, 41), avantageusement par une vis de fixation (33, 43) qui se termine avantageusement à fleur de l'extrémité.

13. Dispositif selon l'une des revendications 1 à 12, **caractérisée en ce que** la vis de col de fémur (2) est montée de façon à pouvoir tourner autour de son axe et l'élément d'arrêt/de freinage glissant (31, 41) exerce dans sa position de fixation une force de maintien respectivement une force de freinage dirigée aussi bien axialement que dans le sens de rotation, de la même manière, sur la vis de col de fémur (2), la portion de glissement étant formée par une portion (200) de la surface d'enveloppe, guidée sur la paroi intérieure du perçage traversant transversal associé (11), de la vis de col de fémur (2).

14. Dispositif selon l'une des revendications 1 à 12, **caractérisé en ce que** la vis de col de fémur (2) est montée de façon à pouvoir tourner autour de son axe et l'élément d'arrêt/de freinage glissant (31, 41) génère dans sa position de fixation une force de maintien axiale respectivement une force de friction à freinage glissant axiale sur la portion de glissement (210) associée à la l'indice de col de fémur (2), tandis que la mobilité en rotation de la vis de col de fémur (2) n'est au moins sensiblement pas influencée.

15. Dispositif selon la revendication 14, **caractérisé en ce que** la portion de glissement (210), associée à la vis de col de fémur (2), est formée par une broche de col de fémur (21) à l'extrémité distale de laquelle la vis de col de fémur (2) est montée de façon à pouvoir tourner autour de son axe, la broche de col de fémur (21) étant guidée en translation, conjointement avec la vis de col de fémur (2), dans un perçage traversant transversal (12) du clou de verrouillage (1) et pouvant être insérée par son extrémité proximale (26) dans le col du fémur.

16. Dispositif selon l'une des revendications 1 à 12, **caractérisé en ce que** la portion de glissement (210), associée à la vis de col de fémur (2), est formée par une broche de col de fémur (21) à l'extrémité distale de laquelle la vis de col de fémur (2) est reliée fixe sans pouvoir tourner, la broche de col de fémur (21) étant guidée en translation, conjointement avec la vis de col de fémur (2), dans un perçage traversant transversal (12) du clou de verrouillage (1) et pouvant être insérée par son extrémité proximale (26) dans le col du fémur.
